# EUROPEAN PATENT APPLICATION

(11) **EP 4 715 333 A1**
(43) Date of publication of application: **25.03.2026**
(21) Application number: 25198417.5
(22) Date of filing: 27.08.2025
(51) Int. Cl.: G01C 22/00, A61B 5/00

(54) **MEASURING DEVICE, MEASURING METHOD, AND PROGRAM**

(30) Priority: 20.09.2024 JP 2024163846
(71) Applicant: Casio Computer Co., Ltd., Tokyo 151-8543 (JP)
(72) Inventor: Matsumoto, Naoya, Hamura-shi, Tokyo, 205-8555 (JP)
(74) Representative: Grünecker Patent- und Rechtsanwälte PartG mbB

(57) **Abstract**

A measuring device (100) includes at least one processor (11). The at least one processor acquires triaxial acceleration data for three axes output by a triaxial acceleration sensor attached to a user. The at least one processor estimates the number of steps of the user, based on resultant acceleration data calculated from the triaxial acceleration data. The at least one processor estimates the number of arm swings of the user, based on specific axis acceleration data corresponding to a direction in which an arm of the user is swung among the triaxial acceleration data. The at least one processor measures the number of steps of the user by using the estimated number of steps of the user and the estimated number of arm swings of the user in a switching manner in accordance with a predetermined criterion.

## Description

This application relates to a measuring device, a measuring method, and a program.

At present, a measuring device that measures the number of steps during walking or running performed by a user, based on output of an acceleration sensor attached on the user is known. For example, Unexamined Japanese Patent Application Publication No. 2020-101849 describes a measuring device that measures, when a time duration of a partial waveform having positive acceleration in an acceleration waveform falls within a reference range, the number of steps for one step with respect to the partial waveform.

In the above-described measuring device, the reference range is adjusted according to a state of the user to improve measurement accuracy. Specifically, in the above-described measuring device, when the state of the user is a state of running, a small value is set as a lower limit of the reference range, and when the state of the user is a state of walking, a large value is set as the lower limit of the reference range.

However, as described in Unexamined Japanese Patent Application Publication No. 2020-101849, there is a possibility that simply adjusting the above-described reference range based on the state of the user does not ensure sufficient measurement accuracy. For example, there is a possibility that when the user widely swings the arms, a noise due to the arm swing of the user is included in the acceleration waveform and measurement of the number of steps from the acceleration waveform cannot be appropriately performed. Thus, a technology to improve accuracy of the measurement of the number of steps is expected.

One of advantages of the present disclosure is to provide a measuring device, a measuring method, and a program that improve accuracy of measurement of the number of steps.

In order to achieve the above-described objective, a measuring device according to the present disclosure includes
at least one processor configured to
acquire triaxial acceleration data for three axes output by a triaxial acceleration sensor attached to a user,
estimate a number of steps of the user, based on resultant acceleration data calculated from the triaxial acceleration data,
estimate a number of arm swings of the user, based on specific axis acceleration data corresponding to a direction in which an arm of the user is swung among the triaxial acceleration data, and
measure a number of steps of the user by using an estimated number of steps of the user and an estimated number of arm swings of the user in a switching manner in accordance with a predetermined criterion.

According to the present disclosure, it is possible to improve accuracy of the measurement of the number of steps.

A more complete understanding of this application can be obtained when the following detailed description is considered in conjunction with the following drawings, in which:
FIG. 1 is a configuration diagram of a measuring device according to an embodiment;
FIG. 2 is a diagram illustrating a manner in which the measuring device according to the embodiment is attached on a user;
FIG. 3 is a functional configuration diagram of the measuring device according to the embodiment;
FIG. 4A is a diagram illustrating a resultant acceleration waveform when no specific noise is generated, and FIG. 4B is a diagram illustrating a U-axis acceleration waveform when no specific noise is generated;
FIG. 5A is a diagram illustrating a resultant acceleration waveform when a specific noise is generated, and FIG. 5B is a diagram illustrating a U-axis acceleration waveform when a specific noise is generated;
FIG. 6 is a diagram illustrating a portion of a resultant acceleration waveform after offset processing;
FIG. 7 is a flowchart illustrating step number measurement processing that the measuring device according to the embodiment executes;
FIG. 8 is a flowchart illustrating step number estimation processing illustrated in FIG. 7;
FIG. 9 is a flowchart illustrating arm swing number estimation processing illustrated in FIG. 7; and
FIG. 10 is a diagram illustrating measurement results of measurement error.

An embodiment of the present disclosure is described in detail below with reference to the drawings. Note that the same or equivalent constituent components are designated by the same reference numerals in the drawings.

### Embodiment

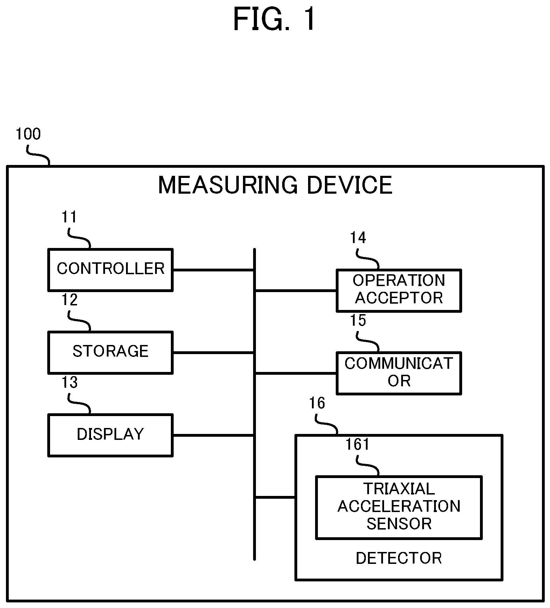

FIG. 1 is a diagram illustrating a configuration of a measuring device 100 according to an embodiment. The measuring device 100 is a device that measures the number of steps due to walking or running by a user. The measuring device 100 is attached to one of the arms of the user and measures the number of steps by detecting acceleration applied to the measuring device 100. The measuring device 100 is attached to a finger, a wrist, an upper arm, or the like. As the measuring device 100, a pedometer dedicated to measurement of the number of steps, a smartwatch having a function of measuring the number of steps, or the like is conceivable. As illustrated in FIG. 1, the measuring device 100 includes a controller 11, a storage 12, a display 13, an operation accepter 14, a communicator 15, and a detector 16.

The controller 11 includes a central processing unit (CPU), a read only memory (ROM), a random access memory (RAM), a real time clock (RTC), and the like. The CPU is also referred to as a central processing device, a central operation device, a processor, a microprocessor, a microcomputer, a digital signal processor (DSP), or the like, and functions as a central operation processor that executes processing and operation relating to control of the measuring device 100. In the controller 11, the CPU controls the measuring device 100 in a unified manner by retrieving a program and data stored in the ROM and using the RAM as a work area. The RTC is, for example, an integrated circuit having a timing function. Note that the CPU is capable of identifying current date and time from time information retrieved from the RTC.

The storage 12 includes a non-volatile semiconductor memory, such as a flash memory, an erasable programmable read only memory (EPROM), and an electrically erasable programmable ROM (EEPROM), and functions as a so-called auxiliary storage device. The storage 12 stores a program and data used by the controller 11 to execute various types of processing. In addition, the storage 12 stores data that the controller 11 generates or acquires by executing various types of processing.

The display 13 displays various types of images in accordance with control performed by the controller 11. For example, the display 13 displays a screen to accept various types of operations from the user. The display 13 includes a touchscreen, a liquid crystal display, or the like. The operation accepter 14 accepts various types of operations from the user and supplies the controller 11 with information indicating details of the accepted operations. The operation accepter 14 includes a touchscreen, a button, a lever, or the like.

The communicator 15 communicates with various types of devices in conformance with a well-known wired communication standard or a well-known wireless communication standard and in accordance with control of controller 11. Examples of the well-known wired communication standard include Universal Serial Bus (USB) (registered trademark), Thunderbolt (registered trademark), and the like. Examples of the well-known wireless communication standard include Wi-Fi (registered trademark), Bluetooth (registered trademark), Zigbee (registered trademark), and the like. The communicator 15 includes a communication interface that conforms to various types of communication standards.

The detector 16 detects acceleration applied to the measuring device 100. The detector 16 includes, for example, a triaxial acceleration sensor 161 that detects acceleration in a U-axis direction, acceleration in a V-axis direction that is orthogonal to the U-axis direction, and acceleration in a W-axis direction that is orthogonal to the U- and V-axis directions. The detector 16 outputs triaxial acceleration data that are time series data of acceleration for three axes. The triaxial acceleration data includes U-axis acceleration data that are time series data of acceleration in the U-axis direction, V-axis acceleration data that are time series data of acceleration in the V-axis direction, and W-axis acceleration data that are time series data of acceleration in the W-axis direction.

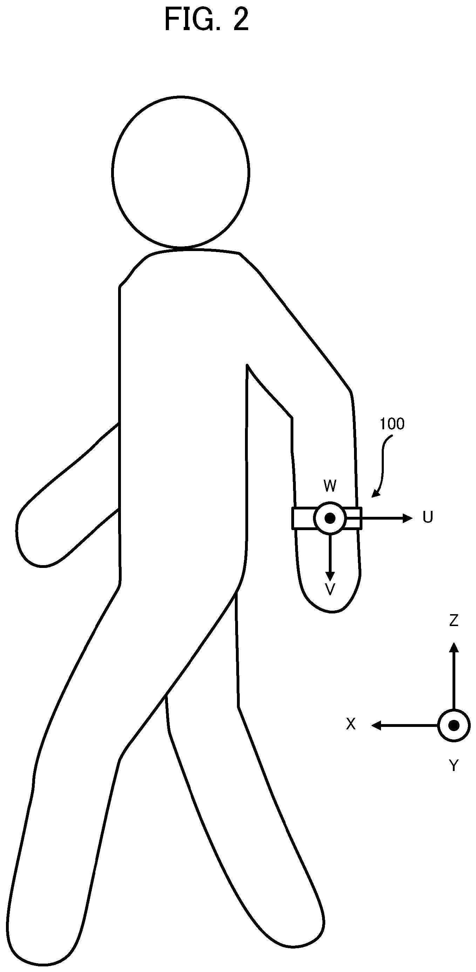

As illustrated in Fig. 2, in the present embodiment, the measuring device 100 is a wristwatch-type device attached to one of the wrists of the user. In the present embodiment, when the measuring device 100 is regarded as an analog watch, the U-axis direction is a direction pointing from a position indicating 6 o'clock to a position indicating 12 o'clock, the V-axis direction is a direction pointing from a position indicating 9 o'clock to a position indicating 3 o'clock, and the W-axis direction is a direction pointing from a contact surface with the wrist to a display surface.

In addition, it is assumed that a direction in which the user moves is an X-axis direction, a direction pointing from a position of the right hand to a position of the left hand is a Y-axis direction, and the vertically upward direction is a Z-axis direction. In this case, the X-axis direction, the Y-axis direction, and the Z-axis direction intersect at right angles with one another. In this configuration, when the measuring device 100 is disposed on the back of the left wrist of the user, the positive direction of the U-axis approximately coincides with the negative direction of the X-axis, the positive direction of the V-axis approximately coincides with the negative direction of the Z-axis, and the positive direction of the W-axis approximately coincides with the positive direction of the Y-axis.

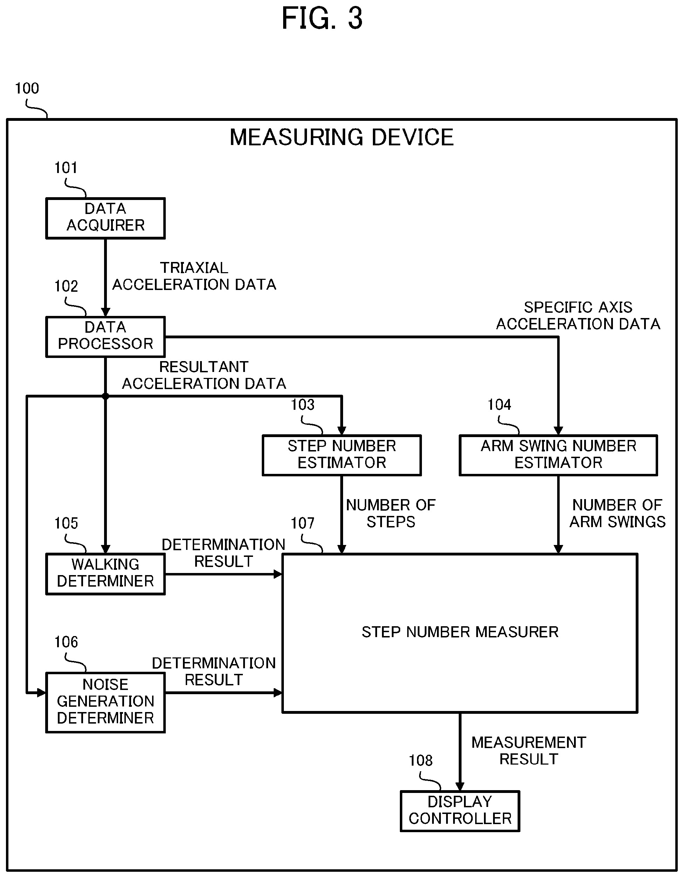

Next, with reference to FIG. 3, functions of the measuring device 100 are described. The measuring device 100 functionally includes a data acquirer 101, a data processor 102, a step number estimator 103, an arm swing number estimator 104, a walking determiner 105, a noise generation determiner 106, a step number measurer 107, and a display controller 108. The foregoing respective functions are achieved by software, firmware, or a combination of software and firmware. The software and the firmware are described as programs and stored in the ROM or the storage 12. The CPU having at least one processor achieves the above-described functions by executing programs stored in the ROM or the storage 12.

The data acquirer 101 acquires triaxial acceleration data output by the triaxial acceleration sensor 161 attached to one the arms of the user. For example, the data acquirer 101 acquires triaxial acceleration data, which are time series data of acceleration for three axes, from the triaxial acceleration sensor 161, which the detector 16 includes. In the present embodiment, a sampling frequency of the triaxial acceleration data is 32 Hz. Therefore, one second of triaxial acceleration data are equivalent to 32 × 3 = 96 pieces of data.

The data processor 102 applies various types of processing to the triaxial acceleration data acquired by the data acquirer 101 and the various types of acceleration data and the like calculated from the triaxial acceleration data. For example, the data processor 102 executes combination processing, filter processing, offset processing, extraction processing, and the like.

The combination processing is processing of combining acceleration for three axes. The combination processing is, for example, processing of calculating a square root of a square sum of acceleration in the U-axis direction, acceleration in the V-axis direction, and acceleration in the W-axis direction with respect to acceleration at each time point and using the calculated square root of the square sum as resultant acceleration. Resultant acceleration data are obtained through the combination processing on triaxial acceleration data.

The filter processing is processing of removing a high frequency component from resultant acceleration, acceleration in one axial direction, or the like. In general, a limit of pitch when a human being walks or runs is approximately 5 Hz. Therefore, in the filter processing, a signal component at 5 Hz or higher is removed as a noise component. For example, when a sampling frequency of acceleration data is 32 Hz, a high frequency component is removed by a low-pass filter the number of taps of which is 7 and the tap coefficients of which are 1, 2, 3, 4, 3, 2, and 1. By applying the filter processing to resultant acceleration data, acceleration data in one axial direction, and the like, acceleration data with a high frequency component removed are acquired.

The offset processing is processing of removing a DC component from resultant acceleration, acceleration in one axial direction, or the like. When the number of steps is to be calculated from change in acceleration due to walking or running or when the number of arm swings is to be calculated from change in acceleration due to arm swing by the user, the DC component of acceleration is unnecessary. Therefore, in the offset processing, the DC component of acceleration is removed as an offset component. For example, when the sampling frequency of acceleration data is 32 Hz, the DC component of acceleration is calculated by an average filter the number of taps of which is 17 and each tap coefficient of which is 1. The calculated DC component is removed from acceleration at each time point. By applying offset processing to resultant acceleration data, acceleration data in one axial direction, and the like, acceleration data with the DC component removed are acquired.

The extraction processing is processing of extracting acceleration data along one axis from triaxial acceleration data. The extraction processing on triaxial acceleration data extracts the U-axis acceleration data, the V-axis acceleration data, or the W-axis acceleration data.

The step number estimator 103 estimates the number of steps of the user during an estimation target period, based on resultant acceleration data calculated from the triaxial acceleration data. As described above, the resultant acceleration data is time series data of resultant acceleration into which acceleration for three axes are combined. The estimation target period is a target period over which the number of steps and the number of arm swings are estimated. In the present embodiment, the estimation target period is a latest one second period, and the number of steps and the number of arm swings are estimated every second.

The arm swing number estimator 104 estimates the number of arm swings of the user in the estimation target period, based on specific axis acceleration data among the triaxial acceleration data. The specific axis acceleration data is time series data of acceleration along a specific axis that corresponds to a direction in which the arm of user is swung. In the present embodiment, the specific axis is the U-axis, and the specific axis acceleration data is the U-axis acceleration data.

The walking determiner 105 determines whether or not the user is walking during the estimation target period, based on the resultant acceleration data. For example, the walking determiner 105 determines whether or not the user is walking, based on an average value of the resultant acceleration, a difference value between a maximum value of the resultant acceleration and a minimum value of the resultant acceleration, and the like.

The noise generation determiner 106 determines whether or not a specific noise is generated during the estimation target period, based on the resultant acceleration data. The specific noise is a noise estimated to be generated caused by arm swing by the user. For example, the noise generation determiner 106 determines whether or not a specific noise is generated, based on the number of local maximum points of the resultant acceleration in a certain period.

The step number measurer 107 measures the number of steps due to walking or running by the user. The step number measurer 107 calculates the number of steps in the estimation target period and adds the calculated number of steps to the cumulative number of steps during a period from a measurement start time point to a start time point of the estimation target period. In the present embodiment, the step number measurer 107 executes the processing of calculating the number of steps in one second and the processing of adding the calculated number of steps to the cumulative number of steps every second. The step number measurer 107 employs the number of steps estimated over the estimation target period or a number double the number of arm swings estimated over the estimation target period, as the number of steps in the estimation target period.

Specifically, when the user is determined not to be walking during the estimation target period or when no specific noise is determined to be generated during the estimation target period, the step number measurer 107 employs the number of steps estimated by the step number estimator 103 as the number of steps of the user in the estimation target period. In contrast, when in a case where the user is determined to be walking during the estimation target period, a specific noise is determined to be generated during the estimation target period, the step number measurer 107 employs a number double the number of arm swings estimated by the arm swing number estimator 104 as the number of steps of the user in the estimation target period.

As described above, the step number measurer 107 employs the number of steps estimated based on the resultant acceleration data when the user is not walking or when no specific noise is generated. In contrast, the step number measurer 107 employs a number double the number of arm swings estimated based on the specific axis acceleration data when the user is walking and a specific noise is generated.

The display controller 108 displays a measurement result measured by the step number measurer 107. For example, the display controller 108 controls the display 13 to display the cumulative number of steps calculated by the step number measurer 107. The cumulative number of steps is an accumulated value of the number of steps measured from the measurement start time point to the current time point.

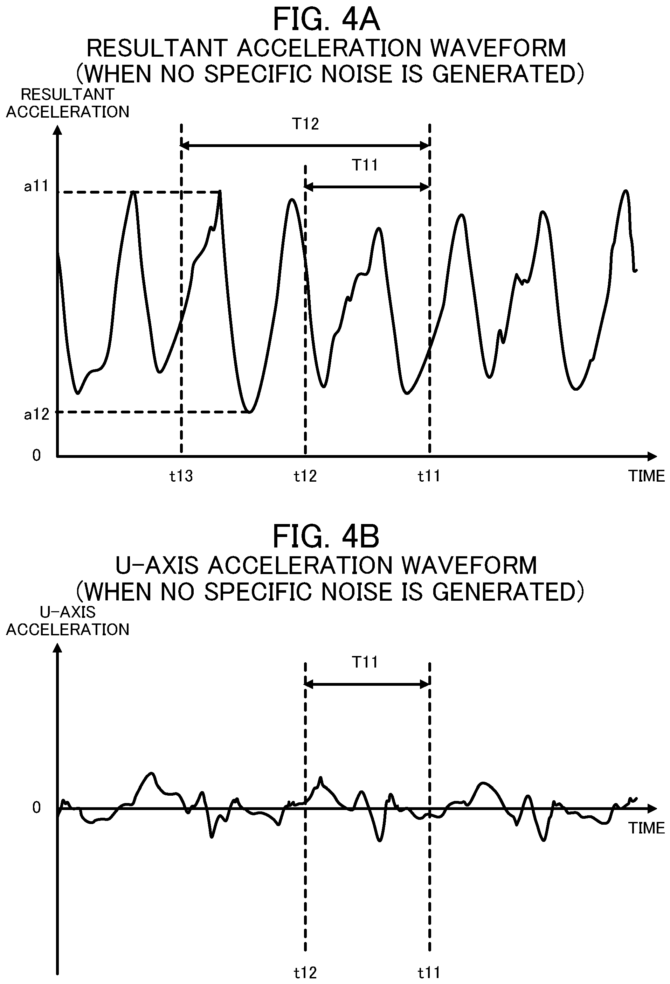

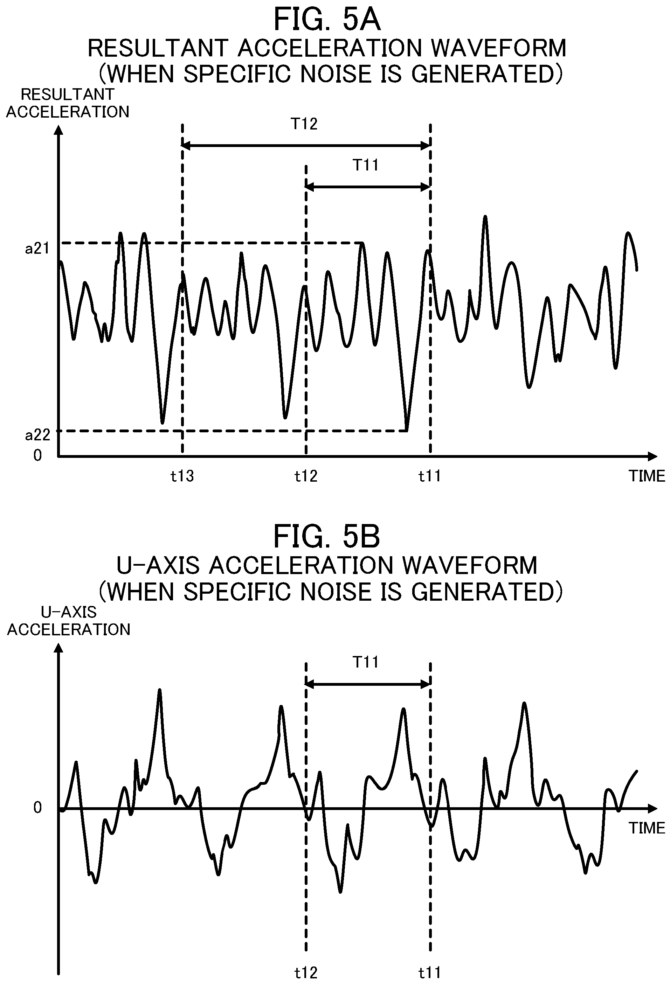

The reason why both the estimation of the number of steps using resultant acceleration data and the estimation of the number of arm swings using specific axis acceleration data are executed is described below with reference to FIGS. 4 and 5.

FIG. 4 is diagrams illustrating acceleration waveforms when no specific noise is generated. More specifically, FIG. 4A is a diagram illustrating a resultant acceleration waveform corresponding to resultant acceleration data after filter processing that are acquired when no specific noise is generated. In addition, FIG. 4B is a diagram illustrating a U-axis acceleration waveform corresponding to U-axis acceleration data after filter processing that are acquired when no specific noise is generated.

FIG. 5 is diagrams illustrating acceleration waveforms when a specific noise is generated. More specifically, FIG. 5A is a diagram illustrating a resultant acceleration waveform corresponding to resultant acceleration data after filter processing that are acquired when a specific noise is generated. In addition, FIG. 5B is a diagram illustrating a U-axis acceleration waveform corresponding to U-axis acceleration data after filter processing that are acquired when a specific noise is generated. In FIGS. 4 and 5, the abscissa is a time axis, and the ordinate is an axis indicating various types of acceleration.

As illustrated in FIG. 4A, in the resultant acceleration waveform when no specific noise is generated, waves with periods corresponding to pitch of walking or running clearly appear. The number of such waves corresponds to the number of steps. Therefore, when no specific noise is generated, it is possible to estimate the number of steps with high accuracy from the resultant acceleration data.

As illustrated in FIG. 4B, in the U-axis acceleration waveform when no specific noise is generated, no wave with a period corresponding to pitch of arm swing appears. Therefore, when no specific noise is generated, it is difficult to estimate the number of arm swings from the U-axis acceleration data.

In contrast, as illustrated by FIG. 5A, in the resultant acceleration waveform when a specific noise is generated, waves corresponding to the specific noise are superimposed on the waves with periods corresponding to the pitch of walking or running, and there is a possibility that waves with periods corresponding to the pitch of walking or running do not clearly appear in the resultant acceleration waveform. Therefore, when a specific noise is generated, there is a possibility that it is difficult to estimate the number of steps from the resultant acceleration data.

As illustrated in FIG. 5B, in the U-axis acceleration waveform when a specific noise is generated, waves with periods corresponding to pitch of arm swing appear. The number of such waves corresponds to the number of arm swings. Therefore, when a specific noise is generated, it is possible to estimate the number of arm swings with high accuracy from the U-axis acceleration.

Therefore, when a specific noise is generated, there is a possibility that it is more suitable to estimate the number of arm swings from the U-axis acceleration data and estimate the number of steps from the estimated number of arm swings, instead of estimating the number of steps from the resultant acceleration data. Note that it is common practice that a user advances two steps with one cycle of arm swing. Therefore, the estimated number of steps is double the estimated number of arm swings.

When the user is running, amplitude of waves with periods corresponding to pitch of running in the resultant acceleration waveform is considered to be large. Thus, even when waves corresponding to the specific noise are superimposed on the waves with periods corresponding to the pitch of running in the resultant acceleration waveform, it is considered that the waves with periods corresponding to the pitch of running clearly appear in the resultant acceleration waveform. In other words, it is considered that when the user is running, the number of steps can be estimated from resultant acceleration waveform data.

In addition, when the user is neither walking nor running, there is a possibility that estimating the number of steps from the number of arm swings estimated from the U-axis acceleration data causes the number of steps to be erroneously measured due to the arm swing. Therefore, when the user is not walking or when no specific noise is generated, it is suitable for the number of steps to be estimated from the resultant acceleration data. In contrast, when the user is walking and a specific noise is generated, it is suitable for the number of steps to be estimated from the number of arm swings estimated from the U-axis acceleration data.

A method for determining whether or not the user is walking can be appropriately adjusted. For example, the walking determiner 105 determines that the user is walking during the estimation target period when in a case where an average value of the resultant acceleration during an average value calculation period falls within an average value reference range, a difference value between a maximum value of the resultant acceleration during a difference value calculation period and a minimum value of the resultant acceleration during the difference value calculation period falls within a difference value reference range.

The average value calculation period is a period over which an average value is calculated and is a period that includes the estimation target period. The difference value calculation period is a period over which a difference value is calculated and is a period that includes the estimation target period. Note that in order to improve determination accuracy, it is suitable for each of the average value calculation period and the difference value calculation period to be a long period to a certain extent. Therefore, in the present embodiment, the average value calculation period and the difference value calculation period are set to a period longer than the estimation target period. Note that in the present embodiment, the difference value calculation period and the average value calculation period are the same period. In FIGS. 4 and 5, T11 denotes the estimation target period, and T12 denotes the average value calculation period and the difference value calculation period. In the present embodiment, length of the average value calculation period and the difference value calculation period is the latest two seconds.

In FIG. 4A, a11 denotes a maximum value of the resultant acceleration during the difference value calculation period, and a12 denotes a minimum value of the resultant acceleration during the difference value calculation period. In this case, the above-described difference value is a difference value between a11 and a12. In FIG. 5A, a21 denotes a maximum value of the resultant acceleration during the difference value calculation period, and a22 denotes a minimum value of the resultant acceleration during the difference value calculation period. In this case, the above-described difference value is a difference value between a21 and a22.

It is considered that the average value of the resultant acceleration when the user is walking is smaller than the average value of the resultant acceleration when the user is running and larger than the average value of the resultant acceleration when the user is at rest. In addition, it is considered that the difference value when the user is walking is smaller than the difference value when the user is running and larger than the difference value when the user is at rest. Therefore, when the average value of the resultant acceleration is a moderate value and the above-described difference value of the resultant acceleration is a moderate value, the user is determined to be walking. The average value reference range is a range within which the average value of the resultant acceleration may fall when the user is walking. The difference value reference range is a range within which the above-described difference value of the resultant acceleration may fall when the user is walking.

A method for determining whether or not a specific noise is being generated can be appropriately adjusted. For example, the noise generation determiner 106 determines that a specific noise is generated when the number of local maximum points of the resultant acceleration in a number calculation period is greater than or equal to a number threshold value. The number calculation period is a period over which the number of local maximum points of the resultant acceleration is calculated and is a period that includes the estimation target period. Note that in order to improve determination accuracy, it is suitable for the number calculation period to be a long period to a certain extent. Therefore, in the present embodiment, the number calculation period is set to a period longer than the estimation target period. Note that in the present embodiment, the difference value calculation period, the average value calculation period, and the number calculation period are the same period.

When no specific noise is generated, the number of local maximum points of the resultant acceleration in the number calculation period corresponds to the number of steps in the number calculation period. In addition, since there is a limit to the pitch at the time of walking, an upper limit of the number of steps in the number calculation period can be identified. Therefore, when the number of local maximum points of the resultant acceleration in the number calculation period is greater than or equal to the number threshold value, a specific noise is determined to be generated during the estimation target period. The number threshold value is a value slightly larger than an upper limit of the number of local maximum values when the user is walking. For example, when the upper limit of the number of steps per second when the user is walking is less than 3, the number threshold value is set to 6.

In the example illustrated in FIG. 4A, three local maximum points are observed in T12, which is the number calculation period. Therefore, when resultant acceleration data corresponding to the resultant acceleration waveform illustrated in FIG. 4A are acquired, it is determined that no specific noise is generated. On the other hand, in the example illustrated in FIG. 5A, nine or ten local maximum points are observed in T12, which is the number calculation period. Therefore, when resultant acceleration data corresponding to the resultant acceleration waveform illustrated in FIG. 5A are acquired, it is determined that a specific noise is generated.

Meanwhile, there is a case where due to a noise generated by a factor other than arm swing, local maximum points the number of which is greater than or equal to the number threshold value are observed in the number calculation period. In this case, it is also considered that the estimation accuracy is low when the number of steps is estimated from resultant acceleration data. Therefore, in this case, the number of steps is estimated from the number of arm swings estimated from the U-axis acceleration data. In other words, the specific noise is not necessarily limited to a noise generated caused by arm swing by the user and may be a noise generated by a factor other than arm swing.

A method in which the step number estimator 103 estimates the number of steps can be appropriately adjusted. For example, the step number estimator 103 estimates the number of steps by counting first partial waveforms that have a time duration falling within a first reference range and an amplitude greater than or equal to a first amplitude threshold value among first partial waveforms included in a resultant acceleration waveform corresponding to resultant acceleration data after shift processing. The first partial waveform is a waveform corresponding to one peak in the resultant acceleration waveform, and is a waveform from a first timing to a second timing within the resultant acceleration waveform.

The first timing is a timing at which the resultant acceleration exceeds an average value of the resultant acceleration. The first timing is a timing at which the resultant acceleration waveform crosses zero in the positive direction. The second timing is a timing at which the resultant acceleration falls below the average value of the resultant acceleration. The second timing is a timing at which the resultant acceleration waveform crosses zero in the negative direction.

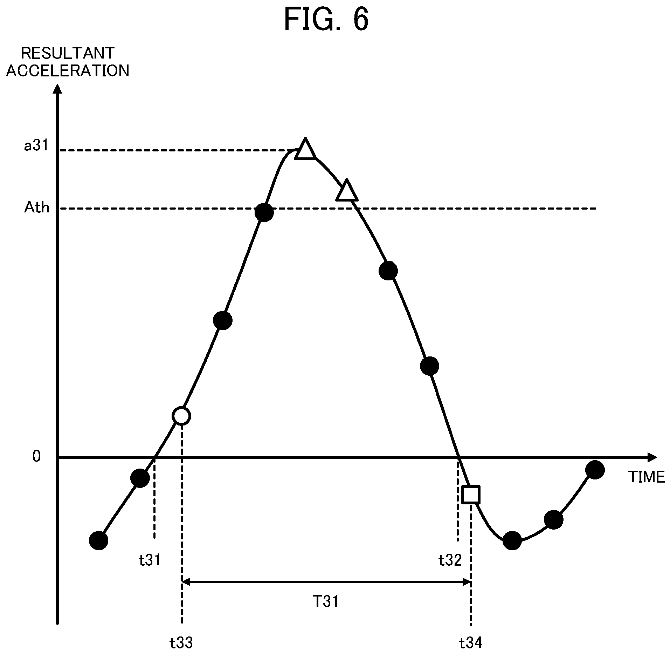

A portion of a resultant acceleration waveform corresponding to resultant acceleration data after shift processing is illustrated in FIG. 6. In FIG. 6, a timing at which the resultant acceleration crosses zero while rising is t31, and a timing at which the resultant acceleration crosses zero while falling is t32. Therefore, it is ideal to set t31 as the first timing and t32 as the second timing. However, when t31 and t32 are to be identified, for example, a processing load associated with interpolation processing is large.

Therefore, in the present embodiment, t33 that is a timing of a first point corresponding to resultant acceleration that has changed from a negative value to a positive value is considered as the first timing. In addition, t34 that is a timing of a second point corresponding to resultant acceleration that has changed from a positive value to a negative value is considered as the second timing. Note that the higher the sampling frequency of the resultant acceleration data is, the smaller a difference between t31 and t33 and a difference between t32 and t34 are. In FIG. 6, the first point is indicated by an open circle, and the second point is indicated by an open square.

When the first partial waveform is a waveform caused by walking or running, the time duration of the first partial waveform corresponds to the pitch of walking or running, and the amplitude of the first partial waveform corresponds to magnitude of acceleration change due to walking or running. A range of the pitch in walking or running is limited to a certain range. In addition, it is considered that acceleration change in walking or running is large to a certain extent.

Therefore, when the time duration of the first partial waveform falls within the first reference range and the amplitude of the first partial waveform is greater than or equal to the first amplitude threshold value, the first partial waveform is considered to be a waveform caused by walking or running, and the number of steps for one step is estimated with respect to the first partial waveform. Note that the first reference range is defined by a first lower limit threshold value and a first upper limit threshold value. On the other hand, when the time duration of the first partial waveform is less than the first lower limit threshold value, the first partial waveform is considered to be a waveform caused by noise. In addition, when the time duration of the first partial waveform exceeds the first upper limit threshold value, the first partial waveform is considered to be a waveform caused by another factor. In addition, when the amplitude of the first partial waveform is less than the first amplitude threshold value, the first partial waveform is considered to be a waveform caused by noise.

In FIG. 6, length of T31 that is a period from t33 to t34 corresponds to the time duration of the first partial waveform. In addition, in FIG. 6, magnitude of a31 that is the maximum value of the resultant acceleration in the first partial waveform corresponds to the amplitude of the first partial waveform. When the length of T31 is the first lower limit threshold value or more and the first upper limit threshold value or less and the magnitude of a31 is greater than or equal to Ath that is the first amplitude threshold value, the first partial waveform is considered to be a waveform caused by walking or running. Note that in FIG. 6, two points indicated by open triangles are points that have resultant acceleration greater than or equal to Ath.

The first lower limit threshold value, the first upper limit threshold value, the first amplitude threshold value, and the like may be predetermined values or may be values that are set according to the state of the user or the like estimated from the resultant acceleration data. For example, the pitch at the time of walking is considered to be longer than the pitch at the time of running. Thus, it is suitable to set the first lower limit threshold value at the time of walking to a larger value than the first lower limit threshold value at the time of running. In this case, it can be expected that a first partial waveform caused by noise is prevented from being considered as a first partial waveform caused by walking, and a first partial waveform caused by running is prevented from being considered as a first partial waveform caused by noise.

In addition, magnitude of acceleration change at the time of walking is considered to be smaller than magnitude of acceleration change at the time of running. Thus, a first amplitude threshold value at the time of walking is suitably set to a smaller value than a first amplitude threshold value at the time of running. In this case, it can be expected that the first partial waveform caused by walking is prevented from being considered as a first partial waveform caused by noise, and the first partial waveform caused by noise is prevented from being considered as a first partial waveform caused by running.

Note that a method for estimating the state of the user from resultant acceleration data can be appropriately adjusted. For example, the pitch at the time of walking is considered to be longer than the pitch at the time of running. Thus, the number of first timings per unit time identified from resultant acceleration data acquired at the time of walking is less than the number of first timings per unit time identified from resultant acceleration data acquired at the time of running. Therefore, when the number of first timings per unit time is less than or equal to the number threshold value, the state of the user may be considered to be a state of walking, and when the number of first timings per unit time exceeds the number threshold value, the state of the user may be considered to be a state of running.

In addition, amplitude at the time of walking is considered to be smaller than amplitude at the time of running. Thus, a root mean square of resultant acceleration identified from resultant acceleration data acquired at the time of walking is smaller than a root mean square of resultant acceleration identified from resultant acceleration data acquired at the time of running. Therefore, when the root mean square of resultant acceleration is less than or equal to a square root threshold value, the state of the user may be considered to be the state of walking, and when the root mean square of resultant acceleration exceeds the square root threshold value, the state of the user may be considered to be the state of running. The state of the user may be determined based on both the number of first timings per unit time and the root mean square of the resultant acceleration.

In addition, a method in which the arm swing number estimator 104 estimates the number of arm swings is basically the same as the method in which the step number estimator 103 estimates the number of steps. For example, the arm swing number estimator 104 can estimate the number of arm swings by counting the number of second partial waveforms that have a time duration falling within a second reference range and amplitude greater than or equal to a second amplitude threshold value among second partial waveforms included in a specific axis acceleration waveform corresponding to specific axis acceleration data after shift processing. The second partial waveform is a waveform corresponding to one peak in a specific axis acceleration waveform, and is a waveform from a third timing to a fourth timing within the specific axis acceleration waveform.

The third timing is a timing at which the specific axis acceleration exceeds an average value of the specific axis acceleration. The third timing is a timing at which the specific axis acceleration waveform crosses zero in the positive direction. The fourth timing is a timing at which the specific axis acceleration falls below the average value of the specific axis acceleration in the specific axis acceleration waveform. The fourth timing is a timing at which the specific axis acceleration waveform crosses zero in the negative direction.

When the second partial waveform is a waveform caused by arm swing, a time duration of the second partial waveform corresponds to the pitch of arm swing, and amplitude of the second partial waveform corresponds to magnitude of acceleration change due to arm swing. A range of the pitch of arm swing is limited to a certain range. In addition, acceleration change in arm swing is considered to be large to a certain extent.

Therefore, when the time duration of the second partial waveform falls within the second reference range and the amplitude of the second partial waveform is greater than or equal to the second amplitude threshold value, the second partial waveform is considered to be a waveform caused by arm swing, and the number of arm swings for one cycle of arm swing is estimated with respect to the second partial waveform. Note that the second reference range is defined by a second lower limit threshold value and a second upper limit threshold value. On the other hand, when the time duration of the second partial waveform is less than the second lower limit threshold value, the second partial waveform is considered to be a waveform caused by noise. In addition, when the time duration of the second partial waveform exceeds the second upper limit threshold value, the second partial waveform is considered to be a waveform caused by another factor. In addition, when the amplitude of the second partial waveform is less than the second amplitude threshold value, the second partial waveform is considered to be a waveform caused by noise. In the present embodiment, the second lower limit threshold value, the second upper limit threshold value, the second amplitude threshold value, and the like, are predetermined values.

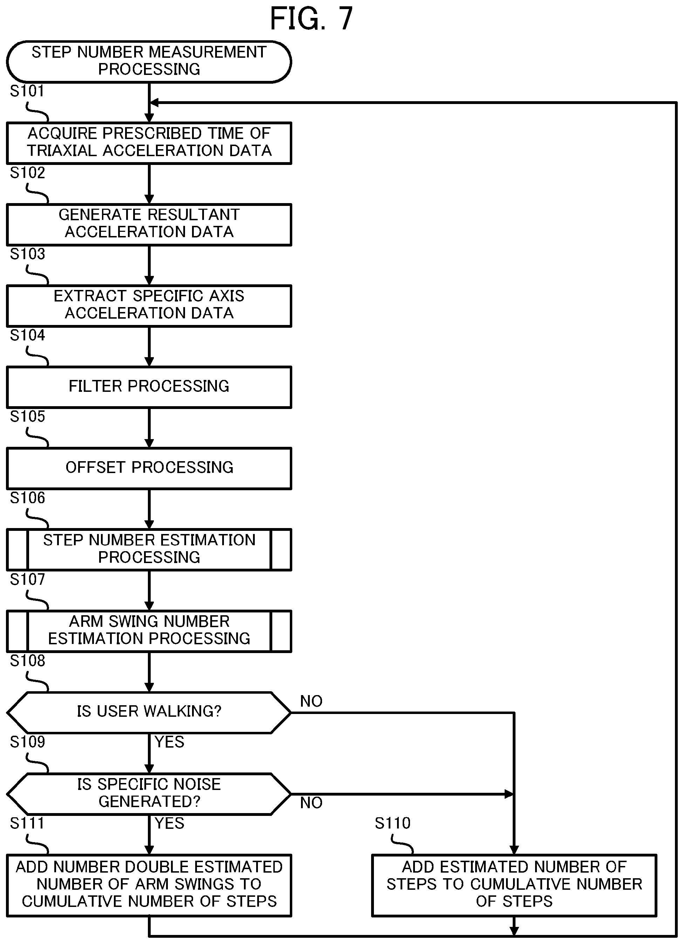

Next, with reference to a flowchart in FIG. 7, step number measurement processing executed by the measuring device 100 is described. The step number measurement processing is, for example, executed in response to the measuring device 100 receiving a start instruction of the step number measurement processing from the user.

First, the controller 11 included in the measuring device 100 acquires a prescribed time of triaxial acceleration data (step S101). For example, the controller 11 acquires one second of triaxial acceleration data that are newly acquired from the triaxial acceleration sensor 161 included in the detector 16. When the controller 11 completes the processing in step S101, the controller 11 generates resultant acceleration data (step S 102). In other words, the controller 11 executes combination processing on the newly acquired one second of triaxial acceleration data and thereby newly generates one second of resultant acceleration data.

When the controller 11 completes the processing in step S102, the controller 11 extracts specific axis acceleration data (step S 103). For example, the controller 11 extracts additional one second of Z-axis acceleration data from the newly acquired one second of triaxial acceleration data. When the controller 11 completes the processing in step S103, the controller 11 executes the filter processing (step S104). For example, the controller 11 applies a low-pass filter to the newly acquired one second of resultant acceleration data and thereby acquire one second of resultant acceleration data with a high frequency component removed. In addition, the controller 11 applies the low-pass filter to the newly acquired one second of specific axis acceleration data and thereby acquire one second of specific axis acceleration data with a high frequency component removed.

When the controller 11 completes the processing in step S104, the controller 11 executes the offset processing (step S105). For example, the controller 11 executes the offset processing on the one second of resultant acceleration data with a high frequency component removed and thereby acquires one second of resultant acceleration data with a DC component removed. In addition, the controller 11 executes the offset processing on the one second of specific axis acceleration data with a high frequency component removed and thereby acquires one second of specific axis acceleration data with a DC component removed.

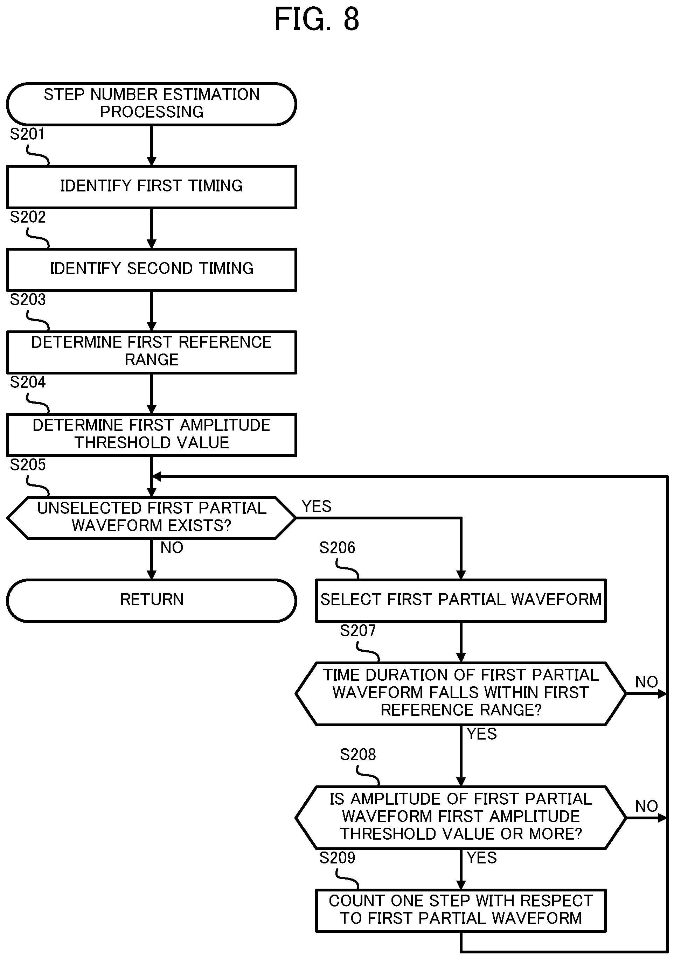

When the controller 11 completes the processing in step S105, the controller 11 executes step number estimation processing (step S106). With reference to FIG. 8, the step number estimation processing is described below in detail.

First, the controller 11 identifies a first timing (step S201). For example, the controller 11 identifies all points at which a resultant acceleration waveform indicated by the one second of resultant acceleration data with a DC component removed changes from a negative value to a positive value. When the controller 11 completes the processing in step S201, the controller 11 identifies a second timing (step S202). For example, the controller 11 identifies all points at which the resultant acceleration waveform indicated by the one second of resultant acceleration data with a DC component removed changes from a positive value to a negative value.

When the controller 11 completes the processing in step S202, the controller 11 determines a first reference range (step S203). For example, the controller 11 estimates a state of the user from the one second of resultant acceleration data with a DC component removed and determines the first reference range, based on the estimated state of the user. When the controller 11 completes the processing in step S203, the controller 11 determines a first amplitude threshold value (step S204). For example, the controller 11 determines the first amplitude threshold value, based on the above-described estimated state of the user.

When the controller 11 completes the processing in step S204, the controller 11 determines whether or not there is an unselected first partial waveform (step S205). In other words, the controller 11 determines whether or not there is a first partial waveform that has not been selected in step S206 among first partial waveforms included in the newly acquired one second of resultant acceleration waveform. Note that selecting a first partial waveform corresponds to selecting a pair of a first timing and a second timing succeeding the first timing.

In this processing, the first partial waveform included in the newly acquired one second of resultant acceleration waveform is a first partial waveform the second timing of which is included in the newly acquired one second of resultant acceleration waveform. In other words, as the first partial waveform included in the newly acquired one second of the resultant acceleration waveform, not only a first partial waveform the first timing and the second timing of which are included in the newly acquired one second of resultant acceleration waveform but also a first partial waveform the first timing of which is included in one second of the resultant acceleration waveform acquired last time and the second timing of which is included in the newly acquired one second of resultant acceleration waveform is conceivable.

When the controller 11 determines that there is no unselected first partial waveform (step S205: NO), the controller 11 completes the step number estimation processing. When the controller 11 determines that there is an unselected first partial waveform (step S205: YES), the controller 11 selects the first partial waveform (step S206). When the controller 11 completes the processing in step S206, the controller 11 determines whether or not the time duration of the first partial waveform falls within the first reference range (step S207). When the controller 11 determines that the time duration of the first partial waveform falls within the first reference range (step S207: YES), the controller 11 determines whether or not the amplitude of the first partial waveform is greater than or equal to the first amplitude threshold value (step S208).

When the controller 11 determines that the amplitude of the first partial waveform is greater than or equal to the first amplitude threshold value (step S208: YES), the controller 11 counts one step with respect to the first partial waveform (step S209). When the controller 11 determines that the time duration of the first partial waveform does not fall within the first reference range (step S207: NO), determines that the amplitude of the first partial waveform is not greater than or equal to the first amplitude threshold value (step S208: NO), or completes the processing in step S209, the controller 11 returns the process to step S205.

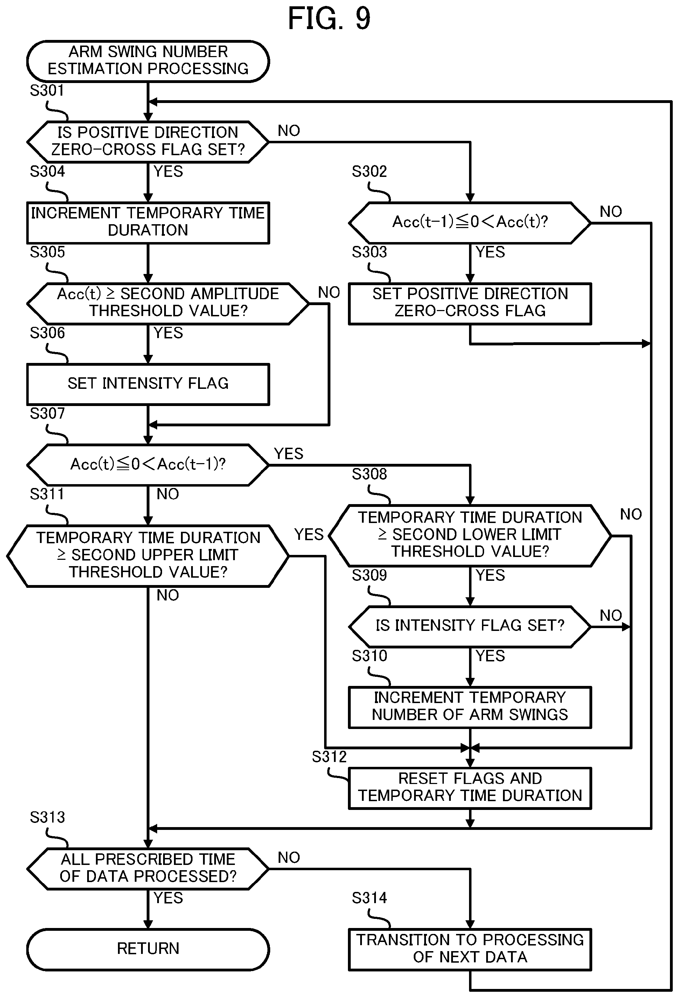

When the controller 11 completes the step number estimation processing in step S106, the controller 11 executes arm swing number estimation processing. With reference to FIG. 9, the arm swing number estimation processing is described below in detail. In the arm swing number estimation processing, newly acquired prescribed time of specific axis acceleration data are processed in order of acquisition time. Note that the specific axis acceleration data is specific axis acceleration data after offset processing.

First, the controller 11 determines whether or not a positive direction zero-cross flag has been set (step S301). The positive direction zero-cross flag is a flag that is set when a zero crossing in the positive direction is detected in a specific axis acceleration waveform indicated by specific axis acceleration data. The detection of a zero crossing in the positive direction corresponds to detection of a third timing. The positive direction zero-cross flag is set in the arm swing number estimation processing performed on a prescribed time of specific axis acceleration data acquired last time or in the arm swing number estimation processing performed on newly acquired prescribed time of specific axis acceleration data.

When the controller 11 determines that the positive direction zero-cross flag has not been set (step S301: NO), the controller 11 determines whether or not Acc(t - 1) ≤ 0 < Acc(t) holds (step S302). Acc(t) denotes data that are currently being processed among the specific axis acceleration data. Acc(t - 1) denotes data that were acquired just before the data that are currently being processed among the specific axis acceleration data. In other words, Acc(t - 1) ≤ 0 < Acc(t) corresponds to detection of a zero crossing in the positive direction in the data currently being processed.

When the controller 11 determines that Acc(t - 1) ≤ 0 < Acc(t) holds (step S302: YES), the controller 11 sets the positive direction zero-cross flag (step S303). When the controller 11 determines that the positive direction zero-cross flag has been set (step S301: YES), the controller 11 increments a temporary time duration (step S304). The temporary time duration is a temporary value of time duration of a second partial waveform. The temporary time duration corresponds to a time from a time point when the data the positive direction zero-cross flag of which is set are acquired to a time point when the data currently being processed are acquired. To increment the temporary time duration corresponds to increasing the temporary time duration by one sampling period.

When the controller 11 completes the processing in step S304, the controller 11 determines whether or not Acc(t) is greater than or equal to a second amplitude threshold value (step S305). In other words, the controller 11 determines whether or not the data currently being processed are greater than or equal to the second amplitude threshold value. When the controller 11 determines that Acc(t) is greater than or equal to the second amplitude threshold value (step S305: YES), the controller 11 sets an intensity flag (step S306). The intensity flag is a flag that is set when specific axis acceleration data indicating the second partial waveform currently being processed include data greater than or equal to the second amplitude threshold value.

When the controller 11 determines that Acc(t) is not greater than or equal to the second amplitude threshold value (step S305: NO) or completes the processing in step S306, the controller 11 determines whether or not Acc(t) ≤ 0 < Acc(t - 1) holds (step S307). Acc(t) ≤ 0 < Acc(t - 1) corresponds to detection of a zero crossing in the negative direction in the data currently being processed. When a zero crossing in the negative direction is detected in the data currently being processed, the second partial waveform currently being processed is completely identified.

When the controller 11 determines that Acc(t) ≤ 0 < Acc(t - 1) holds (step S307: YES), the controller 11 determines whether or not the temporary time duration is greater than or equal to the second lower limit threshold value (step S308). The temporary time duration being greater than or equal to the second lower limit threshold value corresponds to the second partial waveform currently being processed being broad enough not to be assumed as a noise. When the controller 11 determines that the temporary time duration is greater than or equal to the second lower limit threshold value (step S308: YES), the controller 11 determines whether or not the intensity flag has been set (step S309).

When the controller 11 determines that the intensity flag has been set (step S309: YES), the controller 11 increments the temporary number of arm swings (step S310). The temporary number of arm swings is a temporary value of the number of arm swings that is estimated from the newly acquired prescribed time of specific axis acceleration data in the current arm swing number estimation processing. To increment the temporary number of arm swings corresponds to increasing the temporary number of arm swings by one. The temporary number of arm swings is initialized to 0 when the arm swing number estimation processing is started.

When the controller 11 determines that Acc(t) ≤ 0 < Acc(t - 1) does not hold (step S307: NO), the controller 11 determines whether or not the temporary time duration is greater than the second upper limit threshold value (step S311). The temporary time duration being greater than the second upper limit threshold value corresponds to the second partial waveform currently being processed being broad to the extent of not being assumed as a waveform caused by arm swing.

When the controller 11 determines that the temporary time duration is not greater than or equal to the second lower limit threshold value (step S308: NO), determines that the intensity flag has not been set (step S309: NO), completes the processing in step S310, or determines that the temporary time duration is greater than the second upper limit threshold value (step S311: YES), the controller 11 initializes the respective flags and the temporary time duration (step S312). In other words, the controller 11 resets the positive direction zero-cross flag and the intensity flag, and resets the temporary time duration to 0.

When the controller 11 determines that Acc(t - 1) ≤ 0 < Acc(t) does not hold (step S302: NO), determines that the temporary time duration is not greater than the second upper limit threshold value (step S311: NO), or completes the processing in step S303 or S312, the controller 11 determines whether or not all prescribed time of data have been processed (step S313). In other words, the controller 11 determines whether or not the controller 11 has completed processing of all newly acquired prescribed time of specific axis acceleration data.

When the controller 11 determines that the controller 11 has not processed all the prescribed time of data (step S313: NO), the controller 11 transitions to processing of next data (step S314). In other words, the controller 11 increments t that specifies a piece of data to be processed by one. When the controller 11 completes the processing in step S314, the controller 11 returns the process to step S301. When the controller 11 determines that the controller 11 has processed all the prescribed time of data (step S313: YES), the controller 11 completes the arm swing number estimation processing.

When the controller 11 completes the arm swing number estimation processing in step S107, the controller 11 determines whether or not the user is walking (step S108). For example, the controller 11 determines that the user has been walking for the latest one second when in a case where an average value of the resultant acceleration during the latest two seconds falls within the average value reference range, a difference value between the maximum value of the resultant acceleration during the latest two seconds and the minimum value of the resultant acceleration during the latest two seconds falls within the difference value reference range.

When the controller 11 determines that the user is walking (step S108: YES), the controller 11 determines whether or not a specific noise is generated (step S109). For example, the controller 11 determines that a specific noise is generated in the latest one second when the number of local maximum points of the resultant acceleration in the latest two seconds is six or more.

When the controller 11 determines that the user is not walking (step S108: NO) or determines that no specific noise is generated (step S109: NO), the controller 11 adds the estimated number of steps to the cumulative number of steps (step S110). For example, the controller 11 adds an estimated value of the number of steps in the latest one second estimated in the step number estimation processing to the cumulative number of steps.

When the controller 11 determines that a specific noise is generated (step S109: YES), the controller 11 adds a number double the estimated number of arm swings to the cumulative number of steps (step S111). For example, the controller 11 adds a number double the estimated value of the number of arm swings in the latest one second estimated in the arm swing number estimation processing to the cumulative number of steps. When the controller 11 completes the processing in step S110 or S111, the controller 11 returns the process to step S101.

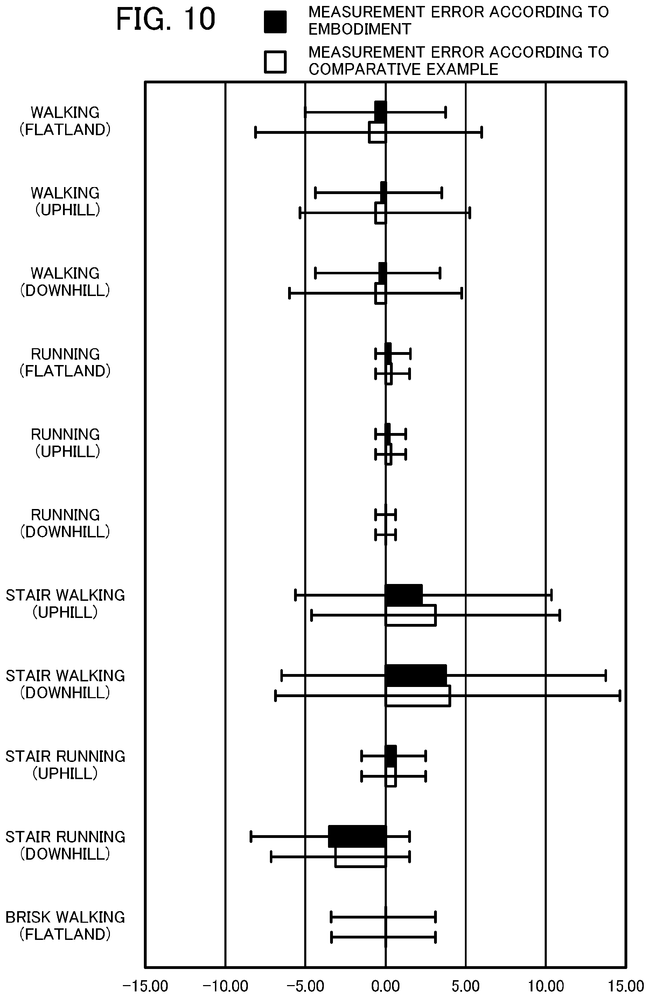

Next, with reference to FIG. 10, error in the measurement of the number of steps using the measuring device 100 is described. FIG. 10 illustrates measurement error when the measurement of the number of steps is performed with respect to various types of trial patterns, using the measuring device 100 and a measuring device according to a comparative example. The measurement error in the measurement of the number of steps is represented by (M-T)/T where M is the number of steps obtained by the measurement of the number of steps and T is the true number of steps, which is expressed as a percentage. A trial pattern is defined by a combination of a classification between walking and running, a location for walking or running, a direction of movement in walking or running involving movement in the up-and-down direction, and the like. In FIG. 10, measurement results when the numbers of steps were measured with respect to 11 trial patterns, using 28 subjects.

In FIG. 10, an average value of error in measurements by the measuring device according to the comparative example is indicated by an open square, and an average value of error in measurements by the measuring device 100 is indicated by a black square. A line laterally extending while passing through an open square or a black square indicates a range of average values of error of the respective subjects. Note that the measuring device 100 executes the step number estimation processing using resultant acceleration data and the arm swing number estimation processing using specific axis acceleration data, whereas the measuring device according to the comparative example executes only the step number estimation processing using resultant acceleration data. In other words, the measuring device 100 measures the number of steps, based on one of an estimation result of the number of steps and an estimation result of the number of arm swings that is estimated to have higher estimation accuracy. In contrast, the measuring device according to the comparative example measures the number of steps, based on an estimation result of the number of steps.

As illustrated in FIG. 10, a result that as a whole, the measurement error associated with the measuring device 100 is smaller than the measurement error associated with the measuring device according to the comparative example was obtained. For example, overall measurement error associated with the measuring device 100 was 0.16 ± 4.00%, and overall measurement error associated with the measuring device according to the comparative example was 0.31 ± 4.44%. In addition, it has been confirmed that in many trial patterns, the measurement error associated with the measuring device 100 involves less variation among the subjects than the measurement error associated with the measuring device according to the comparative example.

Note that larger measurement error associated with the measuring device 100 than measurement error associated with the measuring device according to the comparative example was also confirmed in some trial patterns. For example, it is considered that in a case of stair running (downhill), the measurement error associated with the measuring device 100 is slightly larger than the measurement error associated with the measuring device according to the comparative example. However, with respect to the majority of trial patterns assumed to be performed at the time of measurement of the number of steps, a result that the measurement error associated with measuring device 100 is smaller than the measurement error associated with the measuring device according to the comparative example was obtained. The major trial patterns include walking (flatland), walking (uphill), walking (downhill), running (flatland), running (uphill), running (downhill), and the like. Thus, it is possible to consider that the measurement error associated with the measuring device 100 is essentially smaller than the measurement error associated with the measuring device according to the comparative example.

Conventionally, there has been a possibility that simply adjusting the above-described reference range according to the state of the user does not ensure sufficient measurement accuracy, and, for example, there is a possibility that when the user is widely swinging the arms, noise caused by the arm swing by the user is included in an acceleration waveform, which makes it difficult to appropriately measure the number of steps from the acceleration waveform.

In the present embodiment, when the user is not walking or when no specific noise is generated, the number of steps estimated based on the resultant acceleration data is employed. In contrast, when in a case where the user is walking, a specific noise is generated, a number double the number of arm swings estimated based on specific axis acceleration data is employed as the number of steps of the user. Therefore, according to the present embodiment, it is possible to improve accuracy of the measurement of the number of steps.

In addition, in the present embodiment, the user is determined to be walking when in a case where an average value of resultant acceleration falls within the average value reference range, a difference value between the maximum value of the resultant acceleration and the minimum value of the resultant acceleration falls within the difference value reference range. Therefore, according to the present embodiment, whether or not the user is walking is determined with high accuracy, and it is thus possible to further improve the accuracy of the measurement of the number of steps.

In addition, in the present embodiment, when the number of local maximum points of resultant acceleration in the number calculation period including the estimation target period is greater than or equal to the number threshold value, a specific noise is determined to be generated during the estimation target period. Therefore, according to the present embodiment, whether or not a specific noise is generated is determined with high accuracy, and it is thus possible to further improve the accuracy of the measurement of the number of steps.

In addition, in the present embodiment, when it is determined that the time duration of a first partial waveform within a resultant acceleration waveform falls within the first reference range and the amplitude of the first partial waveform is greater than or equal to the first amplitude threshold value, the number of steps for one step is estimated with respect to the first partial waveform. Therefore, according to the present embodiment, the number of steps is estimated with high accuracy, based on resultant acceleration data, and it is thus possible to further improve the accuracy of the measurement of the number of steps.

In addition, in the present embodiment, when it is determined that time duration of a second partial waveform within a specific axis acceleration waveform falls within the second reference range and the amplitude of the second partial waveform is greater than or equal to the second amplitude threshold value, one cycle of arm swing is estimated with respect to the second partial waveform. Therefore, according to the present embodiment, the number of arm swings is estimated with high accuracy, based on specific axis acceleration data, and it is thus possible to further improve the accuracy of the measurement of the number of steps.

### Variations

Although the embodiment is described above, modification and application in various modes are possible. Which parts of the configuration, functions, and operation described above in the embodiment are to be employed is arbitrarily determined. In addition, in addition to the above-described configuration, functions, and operation, an additional configuration, function, and operation may be employed. In addition, the configuration, functions, and operation described in the above-described embodiment can be freely combined.

The step number estimation processing is not limited to the example described in the embodiment. For example, in the embodiment, an example in which the first reference range, the first amplitude threshold value, and the like are determined according to the state of the user estimated from resultant acceleration data is described. The first reference range, the first amplitude threshold value, and the like may be predetermined values. In addition, in the embodiment, an example in which whether or not to estimate the number of steps for one step with respect to a first partial waveform is determined based on both the time duration of the first partial waveform and the amplitude of the first partial waveform is described. Whether or not to estimate the number of steps for one step with respect to a first partial waveform may be determined based on one of the time duration of the first partial waveform and the amplitude of the first partial waveform. As described above, various types of processing of estimating the number of steps, based on resultant acceleration data can be employed as the step number estimation processing.

Likewise, the arm swing number estimation processing is not limited to the example described in the embodiment. For example, in the embodiment, an example in which the offset processing is executed on specific axis acceleration data is described. The offset processing or the like does not have to be executed on specific axis acceleration data. In addition, in the embodiment, an example in which whether or not to estimate one cycle of arm swing with respect to a second partial waveform is determined based on both the time duration of the second partial waveform and the amplitude of the second partial waveform is described. Whether or not to estimate one cycle of arm swing with respect to a second partial waveform may be determined based on one of the time duration of the second partial waveform and the amplitude of the second partial waveform. As described above, various types of processing of estimating the number of arm swings, based on specific axis acceleration data can be employed as the arm swing number estimation processing.

In the embodiment, an example in which the estimation target period is the latest one second and the average value calculation period, the difference value calculation period, and the number calculation period are the latest two seconds is described. The estimation target period, the average value calculation period, the difference value calculation period, and the number calculation period are not limited to the above-described periods. For example, the estimation target period may be a period less than the latest one second or a period exceeding the latest one second. In addition, the average value calculation period, the difference value calculation period, and the number calculation period may be a period less than the latest two seconds or a period exceeding the latest two seconds. In addition, the average value calculation period, the difference value calculation period, and the number calculation period do not have to be a period including the whole estimation target period. The average value calculation period, the difference value calculation period, and the number calculation period may be periods different from one another.

In the above-described embodiment, the controller 11 functions as respective constituent components illustrated in FIG. 3 by the CPU executing a program stored in the ROM or the storage 12. However, in the present disclosure, the controller 11 may be dedicated hardware. The dedicated hardware is, for example, a single circuit, a composite circuit, a programmed processor, an application specific integrated circuit (ASIC), a field-programmable gate array (FPGA), or a combination of the foregoing. When the controller 11 is dedicated hardware, each of the functions of the constituent components may be achieved by an individual piece of hardware, or the functions of the constituent components may be collectively achieved by a single piece of hardware. In addition, among the functions of the constituent components, some functions may be achieved by dedicated hardware and the other functions may be achieved by software or firmware. As described above, the controller 11 can achieve the above-described functions by hardware, software, firmware, or a combination of the foregoing.

By applying an operation program that defines the operation of the measuring device 100 according to the present disclosure to a computer, such as an existing personal computer or information terminal device, it is possible to cause the computer to function as the measuring device 100 according to the present disclosure. In addition, a method for distributing such a program is arbitrarily determined, and the program may be distributed stored in a non-transitory computer-readable recording medium, such as a compact disk ROM (CD-ROM), a digital versatile disk (DVD), a magneto optical disk (MO), and a memory card, or may be distributed via a communication network, such as the Internet.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

## Claims

1. A measuring device (100) comprising:
at least one processor (11) configured to
acquire triaxial acceleration data for three axes output by a triaxial acceleration sensor (161) attached to a user,
estimate a number of steps of the user, based on resultant acceleration data calculated from the triaxial acceleration data,
estimate a number of arm swings of the user, based on specific axis acceleration data corresponding to a direction in which an arm of the user is swung among the triaxial acceleration data, and
measure a number of steps of the user by using an estimated number of steps of the user and an estimated number of arm swings of the user in a switching manner in accordance with a predetermined criterion.

2. The measuring device (100) according to claim 1, wherein in a case where the at least one processor (11) determines that the user is not walking or that a specific noise is not generated, the at least one processor (11) measures a number of steps of the user by employing the estimated number of steps, and in a case where the at least one processor (11) determines that the user is walking and that the specific noise is generated, the at least one processor (11) measures a number of steps of the user by employing a number double the number of arm swings.

3. The measuring device (100) according to claim 2, wherein in a case where an average value of resultant acceleration during an average value calculation period including an estimation target period falls within an average value reference range and a difference value between a maximum value of resultant acceleration in a difference value calculation period including the estimation target period and a minimum value of resultant acceleration in the difference value calculation period falls within a difference value reference range, the at least one processor (11) determines that the user is walking during the estimation target period.

4. The measuring device (100) according to claim 2 or 3, wherein in a case where a number of local maximum points of resultant acceleration in a number calculation period including an estimation target period is greater than or equal to a number threshold value, the at least one processor (11) determines that the specific noise is generated during the estimation target period.

5. The measuring device (100) according to any one of claims 1 to 3, wherein in a case where the at least one processor (11) determines that a time duration of a first partial waveform falls within a first reference range and amplitude of the first partial waveform is greater than or equal to a first amplitude threshold value, the at least one processor (11) estimates a number of steps for one step with respect to the first partial waveform, the first partial waveform being a waveform from a first timing being a timing at which resultant acceleration exceeds an average value of the resultant acceleration to a second timing being a timing at which the resultant acceleration falls below an average value of the resultant acceleration, in a resultant acceleration waveform corresponding to the resultant acceleration data.

6. The measuring device (100) according to any one of claims 1 to 3, wherein in a case where the at least one processor (11) determines that a time duration of a second partial waveform falls within a second reference range and amplitude of the second partial waveform is greater than or equal to a second amplitude threshold value, the at least one processor (11) estimates one cycle of arm swing with respect to the second partial waveform, the second partial waveform being a waveform from a third timing being a timing at which specific axis acceleration being acceleration along a specific axis exceeds an average value of the specific axis acceleration to a fourth timing being a timing at which the specific axis acceleration falls below an average value of the specific axis acceleration, in a specific axis acceleration waveform corresponding to the specific axis acceleration data.

7. A measuring method in which a computer (11) executes processing comprising:
acquiring triaxial acceleration data for three axes output by a triaxial acceleration sensor (161) attached to a user;
estimating a number of steps of the user, based on resultant acceleration data calculated from the triaxial acceleration data;
estimating a number of arm swings of the user, based on specific axis acceleration data corresponding to a direction in which an arm of the user is swung among the triaxial acceleration data; and
measuring a number of steps of the user by using the estimated number of steps of the user and the estimated number of arm swings of the user in a switching manner in accordance with a predetermined criterion.

8. The measuring method according to claim 7, wherein in a case where the computer (11) determines that the user is not walking or that a specific noise is not generated, the computer (11) measures a number of steps of the user by employing the estimated number of steps, and in a case where the computer (11) determines that the user is walking and that the specific noise is generated, the computer (11) measures a number of steps of the user by employing a number double the number of arm swings.

9. The measuring method according to claim 8, wherein in a case where an average value of resultant acceleration during an average value calculation period including an estimation target period falls within an average value reference range and a difference value between a maximum value of resultant acceleration in a difference value calculation period including the estimation target period and a minimum value of resultant acceleration in the difference value calculation period falls within a difference value reference range, the computer (11) determines that the user is walking during the estimation target period.

10. The measuring method according to claim 8 or 9, wherein in a case where a number of local maximum points of resultant acceleration in a number calculation period including an estimation target period is greater than or equal to a number threshold value, the computer (11) determines that the specific noise is generated during the estimation target period.

11. The measuring method according to any one of claims 7 to 9, wherein in a case where the computer (11) determines that a time duration of a first partial waveform falls within a first reference range and amplitude of the first partial waveform is greater than or equal to a first amplitude threshold value, the computer (11) estimates a number of steps for one step with respect to the first partial waveform, the first partial waveform being a waveform from a first timing, the first timing being a timing at which resultant acceleration exceeds an average value of the resultant acceleration to a second timing being a timing at which the resultant acceleration data falls below an average value of the resultant acceleration, in a resultant acceleration waveform corresponding to the resultant acceleration data.

12. The measuring method according to any one of claims 7 to 9, wherein in a case where the computer (11) determines that a time duration of a second partial waveform falls within a second reference range and amplitude of the second partial waveform is greater than or equal to a second amplitude threshold value, the computer (11) estimates one cycle of arm swing with respect to the second partial waveform, the second partial waveform being a waveform from a third timing being a timing at which specific axis acceleration being acceleration along a specific axis exceeds an average value of the specific axis acceleration to a fourth timing being a timing at which the specific axis acceleration falls below an average value of the specific axis acceleration, in a specific axis acceleration waveform corresponding to the specific axis acceleration data.

13. A program causing a computer (11) to execute processing comprising:
acquiring triaxial acceleration data for three axes output by a triaxial acceleration sensor (161) attached to a user;
estimating a number of steps of the user, based on resultant acceleration data calculated from the triaxial acceleration data;
estimating a number of arm swings of the user, based on specific axis acceleration data corresponding to a direction in which an arm of the user is swung among the triaxial acceleration data; and
measuring a number of steps of the user by using the estimated number of steps of the user and the estimated number of arm swings of the user in a switching manner in accordance with a predetermined criterion.
